# EUROPEAN PATENT APPLICATION

(11) **EP 1 762 215 A1**
(43) Date of publication of application: **14.03.2007**
(21) Application number: 06120290.9
(22) Date of filing: 07.09.2006
(51) Int. Cl.: A61K 8/24, A61Q 11/00

(54) **Oral care composition**

(30) Priority: 13.09.2005 EP 05077091
(71) Applicant: UNILEVER N.V., 3013 AL Rotterdam (NL); UNILEVER PLC, London EC4P 4BQ (GB)
(72) Inventor: Freunscht, Peter, 26841, Casalpusterlengo (IT)
(74) Representative: Newbould, Frazer Anthony

(57) **Abstract**

Oral care composition comprising rod-shaped apatite crystals of the formula Ca₅(PO₄)₃(OH)ₓF_{y}, wherein
a) the length-to-width ratio of the crystals is at least ≥ 5 and
d) x + y = 1, wherein in the case of x or y ≠ 0, the total amount of the crystals is in the form of a mixture of individual hydroxylapatite crystals and fluorapatite crystals and/or in the form of mixed crystals, so that, based on the total amount of the crystals, (1 - x) · 100 % of the hydroxide ions present in the case of y = 0 are replaced by fluoride ions, characterised by:
a surfactant, an abrasive, a humectant and a flavour selected from oils and extracts of basil, camphor, caraway, cardamon, coriander, geranium, ginger, laurel, lavender, mace, menthol, nutmeg, pepper, rose, rosemary, thyme, ylang ylang, jasmin, vanilla, hyssop, lavandin, orris, carrot seed, davana, elemi and osmanthus; borneol and its derivatives; heliotropin, α-ionone, β-ionone, γ-ionone, δ-ionone and derivatives thereof; lactones and thymol, vanillin, ethyl vanillin, maltol and ethyl maltol.

## Description

The invention relates to an oral care composition comprising rod-shaped apatite crystals which have a length-to-width ratio of ≥ 5 and in which the ratio of hydroxide ions to fluoride ions, based on the total amount of the crystals, can be easily varied.

The present invention provides an oral composition according to claim 1.

The rod-shaped apatite crystals used in this invention have a length-to-width ratio of ≥ 5. This means that in the "most adverse" case the crystals have a length-to-width ratio of 5, while there is, however, also a significant number of crystals which have a length-to-width ratio significantly greater than 5, for example 8 to 15.

The rod-shaped hydroxylapatite crystals can made following the disclosure in WO 2003/000588 (BASF).

Preferably, where y ≠ 0, some of the calcium ions are replaced by other cations, in particular sodium, potassium and/or ammonium ions.

Preferably, the length-to-width ratio of the crystals is 5 to 20, preferably 8 to 15, particularly preferably 9 to 12.

Preferably, based on the total amount of the crystals, 0.01 to 30 %, in particular 0.5 to 20 % of the hydroxide ions present in the case of y = 0 are replaced by fluoride ions.

Preferably, the thickness of the individual crystals approximately corresponds to their width.

Preferably, the thickness and the width are 0.01 to 0.02 µm and the length of the crystals is 0.1 to 0.2 µm.

Preferably, the oral care composition is a toothpaste.

The oral care composition according to the invention also comprises a surfactant. Typical surfactants include anionic, nonionic, cationic and zwitterionic or amphoteric surfactants. More preferably, the surfactant is an alkali-metal alkyl sulphate. Most preferably, the surfactant is sodium lauryl sulphate.

The oral care composition according to the invention also comprises an abrasive. Preferred abrasive materials include silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition. Preferred abrasives are chalk and silica, more preferably fine ground natural chalk (FGNC). FGNC provides the optimal formulation window for the rod-shaped crystals since it is much less polar than precipitated calcium carbonate.

The oral care composition according to the invention also comprises a humectant. Preferred humectants include glycerol, sorbitol, propyleneglycol, xylitol, and lactitol.

The oral care composition according to the invention may comprise further ingredients which are common in the art, such as:
antimicrobial agents, e.g. chlorhexidine, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts; anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
binders and thickeners such as sodium carboxymethylcellulose, hydroxyethyl cellulose (Natrosol®), xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

The oil soluble agent is preferably a flavour component. Typical flavour components include those selected from oils and extracts of basil, camphor, caraway, cardamon, coriander, geranium, ginger, laurel, lavender, mace, menthol, nutmeg, pepper, rose, rosemary, thyme, ylang ylang, jasmin, vanilla, hyssop, lavandin, orris, carrot seed, davana, elemi and osmanthus; borneol and its derivatives; heliotropin, α-ionone, β-ionone, γ-ionone, δ-ionone and derivatives thereof; lactones and thymol, vanillin, ethyl vanillin, maltol and ethyl maltol.

Preferably the suspending agent is present in the composition at from 0.001 to 2% by weight, more preferably from 0.1 to 1% by weight and most preferably from 0.25 to 0.75% by weight of the composition.

A preferred optional ingredient is a zinc salt. Preferred zinc salts include zinc citrate, zinc sulphate and zinc fluoride. The most preferred zinc salt is zinc citrate since it provides a synergystic antibacterial effect with the Delmopinol.

A further preferred optional ingredient is Gantrez since it provides a synergystic antibacterial effect with the rod-shaped crystals.

A further preferred optional ingredient is triclosan since it provides a synergystic antibacterial effect with the rod-shaped crystals.

A further preferred optional ingredient is potassium citrate since it provides a synergystic anti-sensitive teeth effect with the rod-shaped crystals.

A further preferred optional ingredient is a fluoride ion source, preferably sodium monofluoro phoshpate or sodium fluoride since it provides a synergystic anti-caries effect with the rod-shaped crystals.

Preferably, the oral care composition according to the invention has a pH of from 5 to 12, more preferably from 7 to 11. Where the composition is a toothpaste and comprises as abrasive silica, the pH of the composition preferably is from 6 to 11. Where the pH is above 9 the buffering is provided by sodium bicarbonate. Where the composition is a toothpaste and comprises as abrasive chalk, more preferably FGNC, the pH of the composition is from 8 to 11, more preferably from 9 to 10.5. These pH conditions provide the best toothpaste composition for the delivery and efficacy of rod-shaped crystals.

### EXAMPLE 1

The following toothpaste is made by standard processes.

| **Ingredient** | **% w/w** |
|---|---|
| WATER DEM. CL. | to 100 |
| NEOSORB 70/70 | 45.00 |
| SODIUM FLUORIDE | 0.32 |
| SODIUM SACCARIN | 0.17 |
| PEG 32 | 5.00 |
| MONOSODIUM PHOSPHATE | 0.10 |
| TITANIUM DIOXIDE | 1.00 |
| CMC 9M | 0.90 |
| TIXOSIL 43 | 7.50 |
| SORBOSIL AC 77 | 10.00 |
| SLS | 1.50 |
| Rod-shaped crystals | 5.00 |
| Flavour | 0.95 |
| | 100.00 |

### EXAMPLE 2

The following experiment compares the seeded crystal growth initiated by the HAP of the invention and that of the prior art, Captal® (ex.Capital Hydroxylapatite).

Fifty milligrams of test and control hydroxylapatite were independently suspended in a remineralising solution (20 mM HEPES, 1.5 mM Ca, 0.9 mM PO₄, pH 7.0) and stirred continuously. Samples were taken and immediately filtered over 24h. The samples were analysed for calcium concentration and neomineralisation was determined by calcium depletion of solutions.

The results are shown in Table 1 where it can be seen that significantly more calcium take-up occurred when the test HAP (ex. BASF) was used than for the Captal® HAP compared with the control HAP (HAP 76).

**Table 1**

| | 0 min | 30 min | 60 min | 1200 min |
|---|---|---|---|---|
| Test HAP | 0 | 0.09 | 0.11 | 0.2 |
| Captal® | 0 | 0.03 | 0.02 | 0.03 |
| HAP76 | 0 | 0.01 | -0.02 | -0.01 |

| | | | | |
|---|---|---|---|---|
| Values = delta Ca (mmol/L) | | | | |

## Claims

1. Oral care composition comprising rod-shaped apatite crystals of the formula Ca₅(PO₄)₃(OH)ₓF_{y}, wherein
a) the length-to-width ratio of the crystals is at least ≥ 5 and
b) x + y = 1, wherein in the case of x or y ≠ 0, the total amount of the crystals is in the form of a mixture of individual hydroxylapatite crystals and fluorapatite crystals and/or in the form of mixed crystals, so that, based on the total amount of the crystals, (1 - x) · 100 % of the hydroxide ions present in the case of y = 0 are replaced by fluoride ions, **characterised by**:
c) a surfactant, an abrasive, a humectant and a flavour selected from oils and extracts of basil, camphor, caraway, cardamon, coriander, geranium, ginger, laurel, lavender, mace, menthol, nutmeg, pepper, rose, rosemary, thyme, ylang ylang, jasmin, vanilla, hyssop, lavandin, orris, carrot seed, davana, elemi and osmanthus; borneol and its derivatives; heliotropin, α-ionone, β-ionone, γ-ionone, δ-ionone and derivatives thereof; lactones and thymol, vanillin, ethyl vanillin, maltol and ethyl maltol.

2. Oral care composition according to claim 1 where y ≠ 0, some of the calcium ions are replaced by other cations, in particular sodium, potassium and/or ammonium ions.

3. Oral care composition according to claim 1 or 2 where the length-to-width ratio of the crystals is 5 to 20, preferably 8 to 15, particularly preferably 9 to 12.

4. Oral care composition according to any preceding claim where, based on the total amount of the crystals, 0.01 to 30 %, in particular 0.5 to 20 % of the hydroxide ions present in the case of y = 0 are replaced by fluoride ions.

5. Oral care composition according to any preceding claim where the thickness of the individual crystals approximately corresponds to their width.

6. Oral care composition according to any preceding claim where the thickness and the width are 0.01 to 0.02 µm and the length of the crystals is 0.1 to 0.2 µm.

7. An oral care composition according to any preceding claim wherein the flavour is present at from 0.001 to 2% by weight of the composition.

8. An oral care composition according to any preceding claim wherein the rod-shaped crystal is present in the composition at from 0.001 to 10% by weight of the composition.

9. An oral care composition according to any preceding claim wherein the surfactant is an alkali-metal alkyl sulphate.

10. An oral care composition according to any preceding claim comprising from 0.75 to 2.5% by weight surfactant.

11. An oral care composition according to any preceding claim comprising from 1 to 65% by weight calcium carbonate.
